# EUROPEAN PATENT SPECIFICATION

(11) **EP 3 744 709 B1**
(45) Date of publication and mention of the grant of the patent: **27.08.2025**
(21) Application number: 18925367.7
(22) Date of filing: 16.07.2018
(51) Int. Cl.: C07C 317/24, C07C 315/06, A01N 41/00, A01P 13/00, A01N 41/10

(54) **NEW CRYSTAL FORM OF TEMBOTRIONE**
NEUE KRISTALLFORM VON TEMBOTRION
NOUVELLE FORME CRISTALLINE DE TEMBOTRIONE

(30) Priority: 06.07.2018 CN 201810734437
(43) Date of publication of application: 02.12.2020
(73) Proprietor: ANHUI JIUYI AGRICULTURE CO., LTD., Hefei, Anhui 230088 (CN); Helm AG, 20097 Hamburg (DE)
(72) Inventor: SHEN, Yunhe, Hefei, Anhui 230088 (CN); STROOT, Joerg, 20097 Hamburg (DE); RAMINHOS, Henrique, 20097 Hamburg (DE)
(74) Representative: Kolster Oy Ab
(86) International application number: PCT/CN2018/095739
(87) International publication number: WO 2020/006778

(56) References cited:
- EP-A1- 2 045 236
- WO-A1-2008/110621
- CN-A- 101 636 383
- CN-A- 101 784 521

## Description

### CROSS REFERENCE TO RELATED APPLICATIONS

This application claims the priority of Chinese Patent Application No. 201810734437.6, filed on July 6, 2018, and titled with "NEW CRYSTAL FORM OF TEMBOTRIONE"

### FIELD

The present disclosure relates to the field of a novel crystal form of herbicide tembotrione, especially to a novel crystal form of tembotrione, a plant protection product therewith and a method for controlling weeds therewith.

### BACKGROUND

Tembotrione is a member of triketone herbicides and belongs to the HPPD-inhibitor herbicides. It was successfully invented by Bayer in 2007. Tembotrione is the youngest member of triketone herbicides. Like other HPPD-inhibitor herbicides, herbicidal activity of tembotrione is expressed by 4-hydroxyphenylpyruvate dioxygenase (HPPD) inhibitors. After HPPD is inhibited, tyrosine accumulates and plastoquinone lacks in weed meristems. After 3-5 days, weeds develop yellowing symptoms, eventually spreading to the entire plant, and weeds become white and die. As a new member of the HPPD herbicides, tembotrione is no less effective in controlling weeds. This herbicide is generally used in combination with the safener isoxadifen-ethyl, which can protect corn from ultraviolet rays, has broad-spectrum, fast-acting properties, and is highly compatible with the environment. Comparing with the well-known mesotrione, tembotrione is not only more active, but also has a wider range of weed control. Tembotrione has a strong killing effect on a variety of weeds, such as thistle genus, convolvulus genus, veronica genus, horseradish genus, urtica genus, chamomile and bedstraw, and weeds that are resistant to glyphosate, dicamba and ALS inhibitor herbicides can also be killed. In addition, tembotrione has a strong ability to resist rain erosion, and can maintain good herbicidal activity throughout the growing season of the crops without causing harm to the next generation of crops.

Different crystal forms of commercially important molecules often have different properties, which can be used in different situations. Therefore, for example, crystalline forms are generally more stable than amorphous forms, so that they can be used for long-term storage of the solid material. Amorphous forms are generally easier to be dissolved than crystalline forms, so that they are more useful than crystalline forms in administration for certain purposes. The crystalline form of a compound affects its physical and chemical properties, such as its melting point, solubility, or dissolution rate. It is therefore advantageous to obtain crystal forms with a certain range of physical and chemical properties, so that the effectiveness can be optimized. Thus in some applications, for example, more stable but less soluble forms may be advantageous, while higher energy, higher solubility forms may provide different benefits in other uses.

Patent WO00/21924 discloses tembotrione (2-[2-chloro-4-methylsulfonyl-3-(2,2,2-trifluoroethoxymethyl)benzoyl]cyclohexane-1,3-dione) and a general method for producing same, which can obtain tembotrione in the form of oil or amorphous solid. Patent WO08110621A and CN101636383 report the crystal forms A, B, and C of tembotrione. EP2045236A1 discloses a solid state form of tembotrione named crystal modification I, II or III, and plant protection products therewith.

### SUMMARY

An object of the present disclosure is to provide a novel crystal form D of tembotrione, which has advantages of higher melting point, better chemical stability, slow dissolution rate, small solubility, and is suitable for long-term storage.

The present disclosure is realized by a following technical solution.

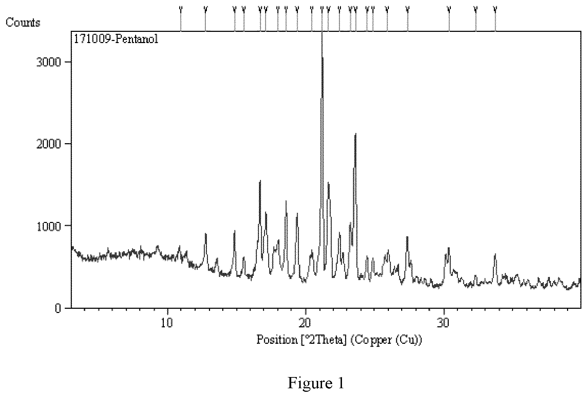

A novel crystal form D of tembotrione, which is confirmed by X-ray powder diffraction method basing on the diffraction pattern thereof. X-ray powder diffraction pattern (Figure 1) recorded with Cu-Kα radiation (1.54443Å) at 25°C shows characteristic reflections represented by 2θ degree or interplanar spacing d (d[Å]), which are shown in Table 1. The crystal form D comprises at least 3, normally at least 5, specially at least 7, and especially all of the following characteristic reflections.

**Table 1 2θ degrees and interplanar spacing d (d[Å]) of crystal form D**

| 2θ | d[Å] |
|---|---|
| 10.9±0.2° | 8.07±0.05 |
| 12.7±0.2° | 6.93±0.05 |
| 14.8±0.2° | 5.96±0.04 |
| 15.5±0.2° | 5.70±0.04 |
| 16.7±0.2° | 5.30±0.04 |
| 17.1±0.2° | 5.17±0.04 |
| 18.0±0.2° | 4.92±0.04 |
| 18.6±0.2° | 4.77±0.04 |
| 19.4±0.2° | 4.57±0.04 |
| 20.4±0.2° | 4.34±0.04 |
| 21.2±0.2° | 4.19±0.04 |
| 21.6±0.2° | 4.10±0.04 |
| 22.5±0.2° | 3.95±0.03 |
| 23.2±0.2° | 3.82±0.03 |
| 23.6±0.2° | 3.77±0.03 |
| 24.4±0.2° | 3.63±0.03 |
| 24.9±0.2° | 3.57±0.03 |
| 25.9±0.2° | 3.43±0.03 |
| 27.3±0.2° | 3.25±0.03 |
| 30.3±0.2° | 2.94±0.02 |
| 32.3±0.2° | 2.77±0.02 |
| 33.7±0.2° | 2.65±0.02 |

In the present disclosure, differential thermogravimetry was also used. The melting point of the novel crystal form of tembotrione was tested with initial temperature of 60°C, the temperature rising to 180°C at a speed of 10°C/min, and maintaining for 10min. The result shows that the melting point of crystal form D of tembotrione is about 124-128°C.

Further, in the present disclosure, the dissolution rate of crystal from D of tembotrione was tested as following: the crystal form D of tembotrione was suspended in a mixture of methanol and water (the volume ratio of methanol : water was 1 : 5) at a temperature of 10-30° C, and the temperature was changed at a rate of 0.5K/min. 14 days later, it was found that the decomposition rate of crystal form D of tembotrione was only 0.06%, and the dissolution rate was slow.

The present disclosure also provides a tembotrione, wherein the tembotrione is basically crystalline, which particularly means containing at least 85% (weight), especially at least 90% (weight), more preferably at least 95% (weight) of crystal form D of tembotrione.

Further, the tembotrione is formed by subjecting tembotrione solid dissolved in a suitable organic solvent or a concentrated reaction solution containing tembotrione to crystallization. Therein, the organic solvent suitable for forming crystal form D is a polar organic solvent.

Preferably, the suitable organic solvent is one selected from ethanol, butanol, pentanol, n-pentanol, acetic acid, acetonitrile, dimethyl sulfoxide, or a mixture thereof.

The crystallization of tembotrione comprises following steps:
(1) Adding tembotrione solid or a concentrated reaction solution containing tembotrione to a suitable organic solvent, stirring to make tembotrione dissolved at a temperature of 10-140°C, especially at least 80°C, giving a solution containing dissolved tembotrione. Due to the temperature for dissolving should not be higher than the boiling point of the solution, further stirring is made at a temperature of 50-120°C to make tembotrione dissolved.
(2) Crystallization: including ① cooling a solution containing dissolved tembotrione to perform crystallization; or ② adding a solvent that can reduce solubility to a solution containing dissolved tembotrione, for example, a non-polar organic solvent or water; or ③ concentrating a solution containing dissolved tembotrione; or any combination of ①-③; or other conventional crystallization methods, etc.

The temperature for crystallization is preferably lower than 120° C, further preferably lower than 90° C, and more preferably lower than 60° C, to achieve a slow crystallization rate.

Further, the cooling rate is 2-8° C/min.

Further, the suitable concentration of the tembotrione solid or the concentrated reaction solution containing tembotrione in the organic solvent is 60-900g/L. The selection of suitable concentration depends on the nature of the organic solvent and solution temperature, and can be determined by those skilled in the art according to specific experiment.

Further, the tembotrione solid is selected from amorphous tembotrione or a mixture thereof, and tembotrione containing other crystal forms or a mixture thereof; for example, a reaction mixture containing tembotrione obtained by chemical reaction (after removal of the reagents and by-products). Reaction and follow-up treatments are performed in an organic solvent or a solvent mixture that is at least partly, preferably at least 40% consisted of solvents suitable for crystallization. The excess reagent and any existing catalyst and any existing unsuitable solvents (such as water, etc.) are removed to obtain a tembotrione mixture containing other crystal forms. The tembotrione solution is prepared by reaction of suitable precursor of tembotrione, etc. The preparation may also be carried out by the methods of the prior art cited in patent WO00/21924 or patent WO008110621A. The other crystal forms herein refer to other crystal forms except for crystal form D.

The present disclosure further provides a plant protection product, which contains the crystal form D of tembotrione.

Further, the plant protection product comprises at least 90wt% of the crystal form D of tembotrione.

The plant protection product according to the present disclosure may be in the form of an aqueous suspension concentrate, a non-aqueous suspension concentrate, or a powder or granule form dispersible in water. The plant protection product can be used on a plant, a ground on which the plant grows, or a seed of the plant, to control the growth of weeds around the plant. Therefore, the present disclosure also provides a method for controlling weeds, comprising spraying the plant protection product to a plant, a ground on which the plant grows, or a seed of the plant.

Compared with the prior art, the present disclosure has one of the following advantages. The present disclosure obtains a novel stable crystal form D, which is unknown before, overcomes the shortages of amorphous tembotrione, has a completely different crystal form compared with crystal forms A, B and C reported in WO08110621A, and makes up the blank of protection of patent of this technology in China. Compared with other crystal forms of tembotrione, crystal form D of tembotrione has advantages of higher melting point, better chemical stability, slow dissolution rate, small solubility, is suitable for long-term storage, and is the crystal structure most suitable for storage of compound tembotrione.

### BRIEF DESCRIPTION OF DRAWINGS

In order to illustrate the technical solutions in the embodiments of the present disclosure or the prior art more clearly, the drawings that are used in the embodiments or the prior art will be briefly introduced hereinafter.
Figure 1 shows the XRPD pattern of crystal form D of tembotrione.

### DETAILED DESCRIPTION

The disclosure discloses a novel crystal form of tembotrione, a method for preparing the same, and use thereof in preparations for plant protection. Those skilled in the art can achieve them in view of the content of this disclosure with appropriate improvement of the process parameters. The product of the present disclosure are described by preferred examples. It is obvious for those skilled in the art to implement or apply the technology of the present disclosure by changing or appropriately modifying and combining the methods described herein without departing from the content, spirit and scope of the present disclosure.

The present disclosure provides a crystalline tembotrione, comprising at least 85% (weight), especially at least 90% (weight), particularly at least 95% (weight) of the crystal form D. The crystal form D can be confirmed by X-ray powder diffraction method based on the diffraction pattern thereof. As shown in Figure 1 and Table 1, an X-ray powder diffraction pattern is recorded with Cu-Kα radiation (1.54443Å) at 25°C, and shows characteristic reflections represented by 2θ degree or interplanar spacing d (d[Å]).

**Table 1 2θ degrees and interplanar spacing d (d[Å]) of the crystal form D**

| 2θ | d[Å] |
|---|---|
| 10.9±0.2° | 8.07±0.05 |
| 12.7±0.2° | 6.93±0.05 |
| 14.8±0.2° | 5.96±0.04 |
| 15.5±0.2° | 5.70±0.04 |
| 16.7±0.2° | 5.30±0.04 |
| 17.1±0.2° | 5.17±0.04 |
| 18.0±0.2° | 4.92±0.04 |
| 18.6±0.2° | 4.77±0.04 |
| 19.4±0.2° | 4.57±0.04 |
| 20.4±0.2° | 4.34±0.04 |
| 21.2±0.2° | 4.19±0.04 |
| 21.6±0.2° | 4.10±0.04 |
| 22.5±0.2° | 3.95±0.03 |
| 23.2±0.2° | 3.82±0.03 |
| 23.6±0.2° | 3.77±0.03 |
| 24.4±0.2° | 3.63±0.03 |
| 24.9±0.2° | 3.57±0.03 |
| 25.9±0.2° | 3.43±0.03 |
| 27.3±0.2° | 3.25±0.03 |
| 30.3±0.2° | 2.94±0.02 |
| 32.3±0.2° | 2.77±0.02 |
| 33.7±0.2° | 2.65±0.02 |

Therein, the crystal form D comprises all of the characteristic reflections.

A method for preparing the above crystalline tembotrione is also disclosed, but not the subject of the invention, the method comprising subjecting tembotrione solid or a concentrated reaction solution containing tembotrione to a suitable organic solvent to crystallization.

Therein, the suitable organic solvent is a polar organic solvent.

Further as preferred, the suitable organic solvent is one selected from ethanol, butanol, pentanol, n-pentanol, acetic acid, acetonitrile, and dimethyl sulfoxide, or a mixture thereof.

The tembotrione solid or concentrated solution containing tembotrione is selected from amorphous tembotrione or a mixture thereof, and tembotrione containing other crystal forms or a mixture thereof; for example, a reaction mixture containing tembotrione obtained by chemical reaction (after removal of the reagents and by-products). Reaction and follow-up treatments are performed in an organic solvent or a solvent mixture that is at least partly, preferably at least 40% consisted of solvents suitable for crystallization. The excess reagent and any existing catalyst and any existing unsuitable solvents (such as water, etc.) are removed to obtain a tembotrione mixture containing other crystal forms. The tembotrione solution can also be prepared by reaction of suitable precursor of tembotrione. These prior art are hereby incorporated by reference.

The temperature for crystallization is preferably lower than 120°C, further preferably lower than 90°C, and more preferably lower than 60°C, to achieve a slow crystallization rate.

The specific steps include the following.
(1) Adding tembotrione solid or a concentrated solution containing tembotrione to a suitable organic solvent, stirring to make tembotrione dissolved at a temperature of 10-140°C, especially at least 50°C, giving a solution containing dissolved tembotrione. Due to the temperature for dissolving should is higher than the boiling point of the solution, further stirring is made at a temperature of 50-120°C to make tembotrione dissolved. Therein, the suitable concentration of the tembotrione solid or concentrated reaction solution containing tembotrione in the organic solvent is preferably 60-900g/L, but is not limited to this. The suitable concentration depends on the nature of the organic solvent and solution temperature, and can be determined by those skilled in the art according to specific experiment.
(2) Crystallization: including ① cooling a solution containing dissolved tembotrione to perform crystallization; or ② adding a solvent that can reduce solubility to a solution containing dissolved tembotrione, for example, a non-polar organic solvent or water; or ③ concentrating a solution containing dissolved tembotrione; or any combination of ①-③; or other conventional crystallization methods, etc.

In order to further understand the present disclosure, the technical solutions in the embodiments of the present disclosure will be clearly and completely described in combination with the examples of the present disclosure. Obviously, the described embodiments are only a part of the embodiments of the present disclosure, but not all the embodiments. Based on the embodiments of the present disclosure, all other embodiments obtained by those skilled in the art without creative efforts should fall within the present disclosure.

Unless otherwise specified, the reagents involved in the examples of the present disclosure are all commercially available products and can be purchased through commercial channels.

### Example 1

160mL n-pentanol was added in a 500ml four-necked flask. Under a condition of stirring, 60g tembotrione of other crystal forms was slowly added in to evenly disperse the material. The mixture was stirred for 5min. The mixture was heated, and the temperature slowly rose to 89°C (the time was about 12min). The material slowly dissolved and the mixture became transparent. The reaction solution was brownish red and transparent kept at 89°C, and thermally insulated for 5min. After the thermal insulation, the temperature was slowly lowered. At 54°C, a small amount of solid material started to separate out. The temperature was continuously lowered to 35°C (the time was about 30min). Ice water was started to be used to continuously lower the temperature to 5°C (the time was about 5min). The temperature was maintained at 5°C and thermal insulation was performed for 40min. A large amount of yellow material separated out. After the thermal insulation, suction filtration was carried out under negative pressure, giving light yellow crystalline material. The material was dried in a 54°C drying oven, weighted, and the content was determined to be 96.5%.

XRPD diffraction pattern of a sample was tested, basically as shown in Figure 1. The obtained light yellow crystalline material was confirmed to be crystal form D of tembotrione.

### Example 2

160mL ethanol was added in a 500ml four-necked flask. Under a condition of stirring, 60g tembotrione of other crystal forms was slowly added in to evenly disperse the material. The mixture was stirred for 5min. The mixture was heated, and the temperature slowly rose to 90°C (the time was about 12min). The material slowly dissolved and the mixture became transparent. The reaction solution was brownish red and transparent kept at 90°C, and thermally insulated for 5min. After the thermal insulation, the temperature was slowly lowered. At 52°C, a small amount of solid material started to separate out. The temperature was continuously lowered to 35°C (the time was about 30min). Ice water was started to be used to continuously lower the temperature to 5°C (the time was about 5min). The temperature was maintained at 5°C and thermal insulation was performed for 40min. A large amount of yellow material separated out. After the thermal insulation, suction filtration was carried out under negative pressure, giving light yellow crystalline material. The material was dried in a 54°C drying oven, weighted, and the content was determined to be 96.05%.

XRPD diffraction pattern of a sample was tested, basically as shown in Figure 1. The obtained light yellow crystalline material was confirmed to be crystal form D of tembotrione.

### Example 3

120mL butanol was added in a 500ml four-necked flask. Under a condition of stirring, 60g tembotrione of other crystal forms was slowly added in to evenly disperse the material. The mixture was stirred for 5min. The mixture was heated, and the temperature slowly rose to 89°C (the time was about 12min). The material slowly dissolved and the mixture became transparent. The reaction solution was brownish red and transparent kept at 90°C, and thermally insulated for 5min. After the thermal insulation, the temperature was slowly lowered. At 60°C, 60 mL cyclohexane was added, and a small amount of solid material started to separate out. The temperature was continuously lowered to 35°C (the time was about 30min). Ice water was started to be used to continuously lower the temperature to 5°C (the time was about 5min). The temperature was maintained at 5°C and thermal insulation was performed for 30min. A large amount of yellow material separated out. After the thermal insulation, suction filtration was carried out under negative pressure, giving light yellow crystalline material. The material was dried in a 54°C drying oven, weighted, and the content was determined to be 96.2%.

XRPD diffraction pattern of a sample was tested, basically as shown in Figure 1. The obtained light yellow crystalline material was confirmed to be crystal form D of tembotrione.

### Example 4

200mL n-pentanol was added in a 500ml four-necked flask. Under a condition of stirring, 100g concentrated tembotrione reaction solution was slowly added in to evenly disperse the material. The mixture was stirred for 5min. The mixture was heated, and the temperature slowly rose to 70°C (the time was about 12min). The material slowly dissolved and the mixture became transparent. The reaction solution was brownish red and transparent kept at 70°C, and thermally insulated for 5min. After the thermal insulation, the temperature was slowly lowered (the room temperature was about 15°C). At 54°C, a small amount of solid material started to separate out. The temperature was continuously lowered to 35°C (the time was about 30min). Ice water was started to be used to continuously lower the temperature to 5°C (the time was about 5min). The temperature was maintained at 5°C and thermal insulation was performed for 40min. A large amount of yellow material separated out. After the thermal insulation, suction filtration was carried out under negative pressure, giving light yellow material. The material was dried in a 54°C drying oven, weighted, and the content was determined to be 96.05%.

XRPD diffraction pattern of a sample was tested, basically as shown in Figure 1. The obtained light yellow crystalline material was confirmed to be crystal form D of tembotrione.

### Example 5 Melting point test

Crystal forms A, B and C were prepared according to the method in patent WO08110621A.

Parameters was set according to differential thermogravimetry: the initial temperature was 60°C, the temperature was raised to 180°C at a speed of 10°C/min, and maintained 10min. The melting points tested for each crystal form were: crystal form A of tembotrione: 119-122°C; crystal form B of tembotrione: 116-119°C; crystal form C of tembotrione: 122-125°C; and crystal form D of tembotrione: 124-128°C.

### Example 6 Dissolution rate test

Crystal forms A, B, C and D of tembotrione were suspended in a mixture of methanol and water (the volume ratio of methanol to water was 1 : 5) at a temperature of 10-30° C, and the temperature was periodically changed at a rate of 0.5K/min. 14 days later, it was found that the decomposition rate of crystal form D of tembotrione was 0.06%, that of crystal form A of tembotrione was 0.1%, that of crystal form B of tembotrione was 0.4%; and that of crystal form C of tembotrione was 0.15%.

### Example 7 Solubility test

At 25±2°C, 100mL (which may be scaled down) solvent was added in a beaker.

A certain mass of crystal forms A, B, C and D of tembotrione were weighted (to ensure insufficient dissolution, tembotrione may be weighted more than once if necessary and the total mass was calculated).

The solute was gradually added, continuously stirred and sufficiently dissolved until the solution was exactly saturated (if there was solid precipitation, it was filtrated out and dried, and put back to the undissolved solid).

Total mass of the undissolved solid was weighted to calculate the mass of the dissolved solid in 100mL (100g) solvent, i.e., the solubility of the solid in the solvent at the temperature.

In the present disclosure, solubility of crystal forms A, B, C and D of tembotrione in several common solvents were tested, and the results were shown in Table 2.

**Table 2 Solubility of crystal forms A, B, C and D of tembotrione in different solvents**

| solvent/ crystal form | A | B | C | D |
|---|---|---|---|---|
| water (PH=4, g/l, 25°C) | 0.26 | 0.28 | 0.25 | 0.22 |
| toluene (g/l, 25°C) | 80.0 | 81.0 | 80.2 | 75.7 |
| methanol (g/l, 25°C) | 8.6 | 8.8 | 8.5 | 8.2 |

The results of Table 2 show that crystal form D of tembotrione has smaller solubility than that of crystal forms A, B and C of tembotrione.

The above is a detailed embodiment and specific operation process of the present disclosure, which is implemented based on the technical solution of the present disclosure.

## Claims

1. A crystal form D of tembotrione, wherein the X-ray powder diffraction pattern of it at 25 °C and Cu-Kα radiation comprises all of the following characteristic reflections with a 2θ degree of 10.9±0.2°, 12.7±0.2°, 14.8±0.2°, 15.5±0.2°, 16.7±0.2°, 17.1±0.2°, 18.0±0.2°, 18.6±0.2°, 19.4±0.2°, 20.4±0.2°, 21.2±0.2°, 21.6±0.2°, 22.5±0.2°, 23.2±0.2°, 23.6±0.2°, 24.4±0.2°, 24.9±0.2°, 25.9±0.2°, 27.3±0.2°, 30.3±0.2°, 32.3±0.2° and 33.7±0.2°.

2. A tembotrione, comprising the crystal form D of tembotrione according to claim 1.

3. The tembotrione according to claim 2, comprising at least 95wt% of crystal form D of tembotrione.

4. A plant protection product, comprising at least 90wt% of the crystal form D of tembotrione according to claim 1.

5. A method for controlling weeds, comprising spraying the plant protection product according to claim 4 to a plant, a ground on which the plant grows, or a seed of the plant.

## Patentansprüche

1. Kristallform D von Tembotrion, wobei das Röntgenpulverbeugungsdiagramm davon bei 25°C and Cu-Kα-Strahlung alle der folgenden charakteristischen Reflexionen mit einem 2θ-Grad von 10,9 ± 0,2°, 12,7 ± 0,2°, 14,8 ± 0,2°, 15,5 ± 0,2°, 16,7 ± 0,2°, 17,1 ± 0,2°, 18,0 ± 0,2°, 18,6 ± 0,2°, 19,4 ± 0,2°, 20,4 ± 0,2°, 21,2 ± 0,2°, 21,6 ± 0,2°, 22,5 ± 0,2°, 23,2 :t 0,2°, 23,6 ± 0,2°, 24,4 ± 0,2°, 24,9 ± 0,2°, 25,9 ± 0,2°, 27,3 ± 0,2°, 30,3 ± 0,2°, 32,3 ± 0,2° und 33,7 ± 0,2° umfasst.

2. Tembotrion, umfassend die Kristallform D von Tembotrion nach Anspruch 1.

3. Tembotrion nach Anspruch 2, umfassend mindestens 95 Gew.-% Kristallform D von Tembotrion.

4. Pflanzenschutzprodukt, umfassend mindestens 90 Gew.-% der Kristallform D von Tembotrion nach Anspruch 1.

5. Verfahren zur Unkrautbekämpfung, umfassend ein Sprühen des Pflanzenschutzprodukts nach Anspruch 4 auf eine Pflanze, einen Erdboden, auf dem die Pflanze wächst, oder ein Saatgut der Pflanze.

## Revendications

1. Forme cristalline D de tembotrione, dans laquelle le motif de diffraction des rayons X par la technique des poudres de celle-ci à 25°C avec rayonnement Cu-Kα comprend toutes les réflexions caractéristiques suivantes avec un degré 2θ de 10,9 ± 0,2°, 12,7 ± 0,2°, 14,8 ± 0,2°, 15,5 ± 0,2°, 16,7 ± 0,2°, 17,1 ± 0,2°, 18,0 ± 0,2°, 18,6 ± 0,2°, 19,4 ± 0,2°, 20,4 ± 0,2°, 21,2 ± 0,2°, 21,6 ± 0,2°, 22,5 ± 0,2°, 23,2 ± 0,2°, 23,6 ± 0,2°, 24,4 ± 0,2°, 24,9 ± 0,2°, 25,9 ± 0,2°, 27,3 ± 0,2°, 30,3 ± 0,2°, 32,3 ± 0,2° et 33,7 ± 0,2°.

2. Tembotrione comprenant la forme cristalline D de tembotrione selon la revendication 1.

3. Tembotrione selon la revendication 2, comprenant au moins 95 % en poids de la forme cristalline D de tembotrione.

4. Produit phytoprotecteur, comprenant au moins 90 % en poids de la forme cristalline D de tembotrione selon la revendication 1.

5. Méthode de lutte contre les mauvaises herbes, comprenant la pulvérisation du produit phytoprotecteur selon la revendication 4 sur une plante, sur le sol sur lequel pousse la plante, ou sur une graine de la plante.
